# EUROPÄISCHE PATENTANMELDUNG

(11) **EP 0 884 298 A2**
(43) Veröffentlichungstag der Anmeldung: **16.12.1998**
(21) Anmeldenummer: 98109998.9
(22) Anmeldetag: 02.06.1998
(51) Int. Cl.: C07C 43/10, C11D 1/825, C11D 1/83, C11D 1/72, B01F 17/42, D06M 13/17

(54) **Gemini-Tenside, Tensidgemisch und Reinigungsmittel**

(30) Priorität: 12.06.1997 DE 19724897
(71) Anmelder: Henkel Kommanditgesellschaft auf Aktien, 40589 Düsseldorf-Holthausen (DE)
(72) Erfinder: Hees, Udo, Dr., 47269 Duisburg (DE); Block, Christian, Dr., 50733 Köln (DE); Raths, Hans-Christian, Dr., 40789 Monheim (DE)

(57) **Zusammenfassung**

Die Erfindung betrifft Gemini-Tenside und solche Tenside enthaltende Tensidgemische, ihre Verwendung in Reinigungsmitteln, insbesondere HGSM, und Reinigungsmittel, insbesondere HGSM, enthaltend solche Gemini-Tenside und Tensidgemische.

## Beschreibung

Die Erfindung betrifft Gemini-Tenside und Tensidgemische, enthaltend sogenannte "Gemini-Tenside". Weiterhin betrifft die Erfindung die Verwendung von Gemini-Tenside enthaltenden Tensidgemischen in Reinigungsmittein, insbesondere in HGSM, und Reinigungsmittel, insbesondere HGSM, mit einem Gehalt: Gemini-Tensiden.

Unter Gemini-Tensiden werden im Rahmen des vorliegenden Textes Verbindungen verstanden, die mindestens zwei hydrophile Gruppen und mindestens zwei hydrophobe Gruppen pro Molekül aufweisen.

Gemini-Tenside sind beispielsweise aus M.Rosen, Chemtech (1993), S.20 ff; F. Menger, C.A.Litau, J. Am. Chem. Soc. 115 (1993), S. 1083 ff., bekannt. Die in den genannten Literaturstellen beschriebenen Tenside zeichnen sich durch eine ungewöhnlich geringe kritische Mizellenkonzentration und die Fähigkeit aus, die Oberflächenspanung des Wassers stark zu verringern.

Reinigungsmittel, beispielsweise Waschmittel, und insbesondere HGSM weisen oft den Nachteil auf, daß bei zu geringer Dosierung der Mittel oder zu geringer Temperatur der Spül- oder Reinigungsflotte kein befriedigendes Spül- oder Reinigungsergebnis erzielt wird. Dies ist häufig darauf zurückzuführen, daß die im Reinigungsmittel und insbesondere im HGSM enthaltenen Tenside unter den Anwendungsbedingungen keine ausreichende Reinigungskraft mehr aufweisen.

Die WO-A 96/23768 beschreibt die Herstellung von Gemini-Tensiden aus Dimer- und Trimeralkoholen. Eine Kombination der so erhältlichen Gemini-Tenside mit anderen Tensiden in Reinigungsmitteln oder HGSM wird nicht erwähnt.

Häufig sind bekannte Gemini-Tenside in der Herstellung aufwendig und teuer. Die WO-A 96/16033 betriff Gemini-Tenside, hergestellt aus Epoxiden und Polyolen. Gemini-Tenside, deren á-C-Atom des ursprünglich eingesetzten Epoxids nach der Umsetzung mit dem Polyol eine CH₂-Gruppe ausbildet, und die anschließend mit mindestens einem Mol Alkoxid pro verbleibender OH-Gruppe alkoxyliert werden und anschließend sulfatiert werden, werden nicht offenbart. Ebenfalls wird nichts über deren hervorragende Reinigungskraft ausgesagt.

Aufgabe der Erfindung war es daher, eine Klasse von Gemini-Tensiden zur Verfügung zu stellen, die einfach herstellbar ist, und eine ausgezeichnete Reinigungskraft alleine, oder vorzugsweise im Gemisch mit weiteren Tensiden aufweist.

Eine weitere Aufgabe der Erfindung war es, ein Tensidgemisch zur Verfügung zu stellen, das in Reinigungsmitteln und insbesondere in HGSM eine verbesserte Reinigungskraft zeigt.

Es wurde nun gefunden, daß Verbindungen der allgemeinen Formel I 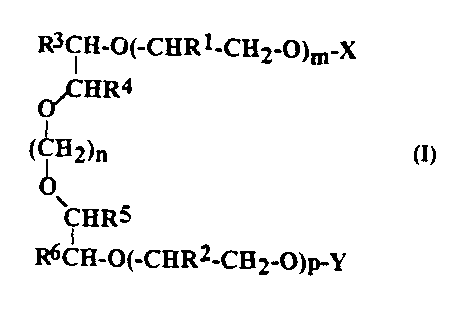 worin R¹ und R² unabhängig voneinander für Wasserstoff und/oder C₁₋₆-Alkyl, R³, R⁴, R⁵ und R⁶ unabhängig voneinander für Wasserstoff oder lineare oder verzweigte Alkylreste mit 4 bis 24 C-Atomen, n für Werte von 2 bis 10, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen stehen, wobei jeweils einer der beiden Reste der Restepaare R³ und R⁴ sowie R⁵ und R⁶ für Wasserstoff stehen muß, leicht herstellbar sind und eine ausgezeichnete Reinigungskraft in Reinigungsmitteln aufweisen.

Gegenstand der Erfindung ist daher eine Verbindung der allgemeinen Formel I, worin R¹ und R² unabhängig voneinander für Wasserstoff und/oder C₁₋₆-Alkyl, R³, R⁴, R⁵ und R⁶ unabhängig voneinander für Wasserstoff oder lineare oder verzweigte Alkylreste mit 4 bis 24 C-Atomen, n für Werte von 2 bis 10, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen stehen, wobei bei den Restepaaren R³ und R⁴ sowie R⁵ und R⁶ jeweils einer der beiden Reste für Wasserstoff stehen muß.

Der Ausdruck "Wasserstoff und/oder C₁₋₆-Alkyl" bedeutet im Rahmen der vorliegenden Erfindung, daß von der allgemeinen Formel I auch Verbindungen mitumfaßt werden, die gemischte Polyetherketten tragen. Gemischte Polyetherketten lassen sich erzeugen, indem eine Alkoxylierung mit wenigstens zwei verschiedenen Alkoxiden, beispielsweise Ethylenoxid (EO) und Propylenoxid (PO), entweder blockweise oder gemischt vorgenommen wird. So entstehen entweder Polyetherketten mit definiert aufeinanderfolgenden Blöcken von Alkoxiden (z.B. EO-EO-EO-PO-PO-PO-EO-EO-EO...) oder statistisch gemischte Ketten (z.B. EO-EO-PO-EO-PO-PO-PO-EO-PO-EO-EO...) Die allgemeine Formel I umfaßt im Rahmen der vorliegenden Erfindung also sowohl Verbindungen mit Polyetherketten, die nur eine Sorte Alkylenoxid enthalten als auch Verbindungen mit Polyetherketten die zwei oder mehr verschiedene Alkylenoxide beinhalten, die blockweise oder statistisch aufeinanderfolgen können.

Es ist besonders bevorzugt, wenn R¹ und R² unabhängig voneinander für Wasserstoff und/oder CH₃ stehen. Es ist weiterhin bevorzugt, wenn R³ oder R⁴ für einen unverzweigten Alkylrest mit mehr als 10 Kohlenstoffatomen steht, wobei der verbleibende Rest für Wasserstoff steht. Es ist ebenfalls bevorzugt, wenn R⁵ oder R⁶ für einen unverzweigten Alkylrest mit mehr als 10 Kohlenstoffatomen steht, wobei der jeweils verbleibende Rest für Wasserstoff steht. Besonder bevorzugt ist es, wenn R³ oder R⁴ für einen unverzweigten Alkylrest mit etwa 12 bis etwa 16 Kohlenstoffatomen steht, wobei der jeweils verbleibende Rest für Wasserstoff steht. Es ist ebenfalls bevorzugt, wenn R⁵ oder R⁶ für einen unverzweigten Alkylrest mit etwa 12 bis etwa 16 Kohlenstoffatomen steht, wobei der jeweils verbleibende Rest für Wasserstoff steht.

Ebenfalls bevorzugt ist es, wenn n für einen Wert von 2 bis 8 steht, beispielsweise 2, 3, 4, 5, 6 oder 7. Weiterhin ist es bevorzugt, wenn m und p unabhängig voneinander für Werte von 3 bis etwa 30, vorzugsweise von etwa 8 bis etwa 20 stehen. Hierbei erkennt der Fachmann sofort, daß es sich gemäß der Natur der Alkoxylierungsreaktion um eine Durchschnittszahl handelt. Einzelne Moleküle können gemäß der entstehenden Homologenverteilung von dieser Durchschnittszahl abweichen. Die Zahl muß demnach auch keine natürliche Zahl sein, es können sich auch gebrochene Zahlen, beispielsweise 8,5, 9,3, 10,7, 11,4, ergeben. In der Regel wird die Durchschnittszahl (bei angenommenem vollständigem Umsatz) dem eingesetzten molaren Verhältnis von Alkoxid zu OH-Gruppen entsprechen. Besonders bevorzugt stehen m und p unabhängig voneinander für Zahlen von etwa 8 bis etwa 12, ganz besonders bevorzugt für etwa 10.

X und Y stehen bevorzugt für SO₃M, wobei M für Wasserstoff oder Alkali- oder Erdalkalimetallionen, insbesondere für Natrium, oder für quaternisierte Stockstoffbasen, beispielsweise Ammoniumionen oder mit gegebenenfalls funktionelle Gruppen (z.B. Hydroxylgruppen) tragenden C₁₋₁₈-Alkylresten quaternisierte Amine, steht.

Die Verbindungen der allgemeinen Formel I lassen sich ausgezeichnet in Reinigungsmitteln, beispielsweise in Waschmitteln, Reinigungsmitteln für harte Oberflächen, Körperreinigungsmitteln, Kosmetika, maschinellen Geschirrspülmitteln, Handgeschirrspülmitteln, als technische Emulgatoren, Netzmittel, Dispergatoren, insbesondere in Lacken und Farben, Demulgatoren, Hydrotrope, Antistatika, Additive für die Erdölindustrie, Kraftstoffadditive, oder in der Metall und Textilverarbeitung verwenden.

Gegenstand der Erfindung ist damit auch die Verwendung von Verbindungen der allgemeinen Formel I in Waschmitteln, Reinigungsmitteln für harte Oberflächen, Körperreinigungsmitteln, Kosmetika, maschinellen Geschirrspülmitteln, Handgeschirrspülmitteln, als technische Emulgatoren, Netzmittel, Dispergatoren, insbesondere in Lacken und Farben, Demulgatoren, Hydrotrope, Antistatika, Additive für die Erdölindustrie, Kraftstoffadditive, oder in der Metall und Textilverarbeitung.

Die durch die allgemeine Formel I bezeichneten Verbindungen gehören zur Klasse der Gemini-Tenside, die im weiteren Verlauf dieses Textes noch näher erläutert werden.

Weiterhin wurde gefunden, daß Gemini-Tenside im Gemisch mit mindestens einem nichtionischen Tensid und mindestens einem anionischen Tensid gegenüber bisher üblichen Reinigungsmittein und insbesondere HGSM eine verbesserte Reinigungskraft zeigen.

Ein weiterer Gegenstand der Erfindung ist daher ein Tensidgemisch, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
   und
b) 0 bis 99 Gew.-% mindestens eines nichtionischen Tensids, das kein Gemini-Tensid ist, als Komponente B
   und/oder
c) 0 bis 99 Gew.-% mindestens eines anionischen Tensids, das kein Gemini-Tensid ist, als Komponente C,
   wobei die Summe der Anteile der Komponenten A, B und C 100 Gew.-% ergibt.

Unter einem "Reinigungsmittel'' wird im Rahmen der vorliegenden Erfindung ein Mittel verstanden, das dem Anwender zunächst in konzentrierter Form vorliegt und zum Gebrauch in Wasser mit einer Temperatur von bis zu etwa 60°C aufgelöst wird. Mit dieser Reinigungsflotte können anschließend Textilien, d harte Oberflächen, wie Glas, Keramik, Beton, Metall, sowie lackierte oder polierte Oberflächen, gereinigt werden.

Die HGSM stellen eine Untergruppe der Reinigungsmittel dar. Zum Gebrauch werden die HGSM in der Regel in Wasser mit einer Temperatur von bis zu etwa 50°C aufgelöst, und die entstehende Spülflotte wird zum Reinigen von Eß- und Kochgeschirr eingesetzt.

Während die in den o.g. Literaturstellen beschriebenen Gemini-Tenside synthetisch nur schwer zugänglich sind, beschreibt die WO-A 96/23768 beschreibt eine einfache Methode zur Herstellung von Gemini-Tensiden aus Dimer- und/oder Trimeralkoholen.

Dimer- und/oder Trimeralkohole sind im Handel erhältliche Verbindungen und können beispielsweise durch Reduktion von Dimer- und/oder Trimerfettsäureestern gewonnen werden, die wiederum z.B. durch Oligomerisierung von ungesättigten Fettsäuren und anschließende Veresterung erhalten werden. Die dabei entstehenden Dimer- und Trimerfettsäuren sind in der Regel Gemische aus acyclischen und cyclischen Dicarbonsäuren mit durchschnittlich 36 bis 44 Kohlenstoffatomen (vgl. A. Hinze, Fette & Öle, 26, (1994)).

Dimer- und/oder Trimeralkoholalkoxylate können in an sich bekannter Weise durch Alkoxylierung der Dimer und/oder Trimeralkohole erhalten werden. Zu den zur Herstellung von Gemini-Tensiden bevorzugt eingesetzten Alkoxylaten gehören beispielsweise die Ethoxylate und die Propoxylate, oder gemischte Alkoxylate, die sowohl Ethoxy- als auch Propoxygruppen enthalten. Besonders bevorzugt sind Addukte mit durchschnittlich 1 bis etwa 20 Ethylenoxidgruppen pro substituierter OH-Gruppe, wobei zusätzlich noch 1 bis etwa 5 Propylenoxidgruppen pro substituierter OH-Gruppe vorhanden sein können. Die angegebenen Alkoxylierungsgrade stellen statistische Mittelwerte dar, die für ein spezielles Produkt eine ganze oder eine gebrochene Zahl sein können. Bevorzugte Dimer- oder Trimeralkoholalkoxylate weisen eine eingeengte Homologenverteilung bezuglich der Zahl der Alkylenoxideinheiten auf (narrow range ethoxylates, NRE).

Die alkoxylierten Dimer- und/oder Trimeralkoholalkoxylate werden mit üblichen Sulfiermitteln, beispielsweise Schwefelsäure, Oleum, Chlorsulfonsäure, Aminosulfonsäure und/oder gasförmigem Schwefeltrioxid, gegebenenfalls im Gemisch mit einem Inertgas, sulfatiert, wobei Chlorsulfonsäure und gasförmiges Schwefeltrioxid bevorzugt sind.

Im Anschluß an die Sulfatierungsreaktion werden die entstandenen Produkte mit einer Base neutralisiert und bevorzugt auf einen pH-Wert unter 9, besonders bevorzugt von etwa 6,5 bis etwa 8,5 eingestellt. Als Basen für die Neutralisation kommen Alkalimetallhydroxide, wie Lithium-, Natrium- oder Kaliumhydroxid, Erdalkalimetalloxide und Erdalkalimetallhydroxide, wie Magnesium- oder Calciumoxid oder -hydroxid, Ammoniak, Mono-, Di- oder Trialkanolamine mit 2 bis 4 Kohlenstoffatomen, sowie primäre, sekundäre oder tertiäre C₁₋₄-Alkylamine, oder Gemische aus 2 oder mehr davon, in Betracht.

Bezüglich der Sulfatierungs- und Neutralisationsreaktion sei nochmals auf die WO-A 96/23768 verwiesen, auf deren Inhalt ausdrücklich Bezug genommen wird.

Die so erhältlichen und im Rahmen der vorliegenden Erfindung eingesetzten Dimer- und/oder Trimeralkoholbis- bzw. -trisethersulfate weisen grenzflächenaktive Eigenschaften auf. Sie fördern beispielsweise die Benetzung fester Oberflächen und die Emulgierung von ansonsten nicht miteinander mischbaren Phasen.

In einer bevorzugten Ausführungsform der Erfindung wird daher als Komponente A mindestens ein Dimeralkohol-bis-ethersulfat oder mindestens ein Trimeralkohol-tris-ethersulfat eingesetzt, das erhältlich ist, indem die Anlagerungsprodukte von Dimer- und/oder Trimeralkoholen und Alkylenoxiden oder Gemische solcher Anlagerungsprodukte mit einem Sulfiermittel umgesetzt werden und anschließend mit einer wäßrigen Base neutralisiert werden.

Ebenfalls als Komponente A einsetzbar sind die Eingangs beschriebenen Verbindungen der allgemeinen Formel I, die beispielsweise aus der ringöffnenden Umsetzung von mindestens 2 Mol epoxidiertem á-Olefin mit etwa 6 bis etwa 26 C-Atomen und 1 Mol Alkylendiol, beispielsweise Ethylenglykol, Propylenglykol, Butandiol-1,4, Pentandiol-1,5, Hexandiol-1,6, Heptandiol-1,7 oder Octandiol-1,8, mit anschließender Alkoxylierung, vorzugsweise Ethoxy-lierung, der entstanden OH-Gruppen und abschließender Sulfatierung, gewonnen werden können.

In einer weiteren bevorzugten Ausführungsform der Erfindung wird daher als Komponente A mindestens eine Verbindung der allgemeinen Formel I eingesetzt, worin R¹ und R² unabhängig voneinander für Wasserstoff und/oder C₁₋₆-Alkyl, R³, R⁴, R⁵ und R⁶ unabhängig voneinander für. Wasserstoff oder lineare oder verzweigte Alkylreste mit 4 bis 24 C-Atomen, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen stehen, wobei bei den Restepaaren R³ und R⁴ sowie R⁵ und R⁶ jeweils einer der beiden Reste für Wasserstoff stehen muß.

Es ist besonders bevorzugt, wenn R¹ und R² unabhängig voneinander für Wasserstoff und/oder CH₃ stehen. Es ist weiterhin bevorzugt, wenn R³ oder R⁴ für einen unverzweigten Alkylrest mit mehr als 10 Kohlenstoffatomen steht, wobei der verbleibende Rest für Wasserstoff steht. Es ist ebenfalls bevorzugt, wenn R⁵ oder R⁶ für einen unverzweigten Alkylrest mit mehr als 10 Kohlenstoffatomen steht, wobei der verbleibende Rest für Wasserstoff steht. Besonder bevorzugt ist es, wenn R³ oder R⁴ für einen unverzweigten Alkylrest mit etwa 12 bis etwa 16 Kohlerstoffatomen steht, wobei der verbleibende Rest für Wasserstoff steht. Es ist ebenfalls bevorzugt, wenn R⁵ oder R⁶ für einen unverzweigten Alkylrest mit etwa 12 bis etwa 16 Kohlenstoffatomen steht, wobei der verbleibende Rest für Wasserstoff steht.

Ebenfalls bevorzugt ist es, wenn n für einen Wert von 2 bis 8 steht, beispielsweise 2, 3, 4, 5, 6 oder 7. Weiterhin ist es bevorzugt, wenn m und p unabhängig voneinander für Werte von 3 bis etwa 30, vorzugsweise von etwa 8 bis etwa 20 stehen. Hierbei erkennt der Fachmann sofort, daß es sich gemäß der Natur der Alkoxylierungsreaktion um eine Durchschnittszahl handelt. Einzelne Moleküle können gemäß der entstehenden Homologenverteilung von dieser Durchschnittszahl abweichen. Die Zahl muß demnach auch keine natürliche Zahl sein, es können sich auch gebrochene Zahlen, beispielsweise 8,5, 9,3, 10,7, 11,4, ergeben. In der Regel wird die Durchschnittszahl (bei angenommenem vollständigem Umsatz) dem eingesetzten molaren Verhältnis von Alkoxid zu OH-Gruppen entsprechen. Besonders bevorzugt stehen m und p unabhängig voneinander für Zahlen von etwa 8 bis etwa 12, ganz besonders bevorzugt für etwa 10.

X und Y stehen bevorzugt für SO₃M, wobei M für Wasserstoff oder Alkali- oder Erdalkalimetallionen, insbesondere für. Natrium steht.

Die Komponente A des erfindungsgemäßen Tensidgemischs kann lediglich ein Gemini-Tensid enthalten, es können jedoch auch Gemische aus zwei oder mehr Gemini-Tensiden als Komponente A eingesetzt werden.

In jedem Fall beträgt der Anteil der Komponente A am erfindungsgemäßen Tensidgemisch etwa 0,1 bis etwa 99 Gew.-%, vorzugsweise etwa 1 bis etwa 98 Gew.-%. Bevorzugt ist es, wenn der Anteil der Komponente A am erfindungsgemäßen Tensidgemisch zwischen etwa 10 Gew.-% und etwa 60 Gew.-%, vorzugsweise zwischen etwa 15 Gew.-% und etwa 55 Gew.-% und besonders bevorzugt zwischen etwa 20 Gew.-% und etwa 50 Gew.-% liegt.

Als Komponente B enthält das erfindungsgemäße Tensidgemisch mindestens ein nichtionisches Tensid, das kein Gemini-Tensid ist.

Typische Beispiele für nichtionische Tenside sind Fettalkoholpolyalkylenether, vorzugsweise mit Ethylenoxid- oder Propylenoxideinheiten, Alkylphenolpolyalkylenether, vorzugsweise mit Ethylenoxid- oder Propylenoxideinheiten, Fettsäurepolyalkylenester, vorzugsweise mit Ethylenoxid- oder Propylenoxideinheiten, Fettsäureamidpolyalkylenether, vorzugsweise mit Ethylenoxid- oder Propylenoxideinheiten, Fettaminpolyalkylenether, vorzugsweise mit Ethylenoxid- oder Propylenoxideinheiten, alkoxylierte Triglyceride, vorzugsweise mit Ethylenoxid oder Propylenoxid alkoxyliert, Aminoxide, Alk(en)yloligoglucoside, Fettsäure-N-alkylglucamide, Proteinhydrolysate (insbesondere pflanzliche Produkte auf Sojabasis), Polyolfettsäureester, Zuckerester, Sorbitanester und Polysorbate. Sofern die nichtionischen Tenside Polyalkylenetherketten enthalten, können sie eine konventionelle, vorzugsweise jedoch eine eingeengte Homologenverteilung aufweisen.

Beispielsweise enthalten die erfindungsgemäßen manuellen Reinigungsmittel oder insbesondere HGSM in der Wirksubstanz als Komponente B mindestens ein Anlagerungsprodukt von Alkylenoxiden an lineare, aliphatische C₈₋₁₆-Alkohole. Als Alkylenoxide sind Ethylenoxid und/oder Propylenoxid bevorzugt. Besonders bevorzugt ist Ethylenoxid, es können jedoch auch Verbindungen mit gemischten Etherketten eingesetzt werden, die sowohl Ethylenoxid als auch Propylenoxid, entweder in zufälliger Reihenfolge oder als sequentielle Blocks, enthalten.

Typische Beispiele für Fettalkohole sind Capronalkohol, Caprylakohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Behenylakohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkoholmischungen, wobei die enthaltenen Fettalkohole etwa 12 bis etwa 18 Kohlenstoffatome aufweisen, beispielsweise Kokos-, Palm-, Palmkern- oder Talgfettalkohole, insbesondere das Ethoxylierungsprodukt von Kokosfettalkoholen, wobei die Alkohole im Durchschnitt etwa 7 Ethylenoxideinheiten aufweisen.

Ebenso als Komponente B einsetzbar sind die Amide von Alkylcarbonsäuren, vorzugsweise von Alkylcarbonsäuren mit etwa 6 bis etwa 24 C-Atomen, mit Alkanolamiden, vorzugsweise Monoalkanolamiden. Besonders bevorzugt sind hierunter die Amide, die aus natürlichen oder synthetisch hergestellten Fettsäuren und Fettsäureschnitten mit Aminoethanol erhältlich sind, ganz besonders bevorzugt sind dabei die Monoethanolamide aus Kokosfettsäureschnitten, insbesondere den C₈₋₁₄-Fettsäureschnitten und Ethanolamin.

Bevorzugt wird als Komponente B mindestens ein Alkylpolyglykosid oder mindestens ein Fettsäureglucamid eingesetzt. Das entsprechende nichtionische Tensid kann im erfindungsgemäßen Reinigungsmittel, insbesondere im HGSM, beispielsweise als Netzmittel oder zur besseren Ablösung fetthaltiger Substanzen dienen.

Besonders bevorzugt wird als Komponente B mindestens ein Alkylpolyglykosid der allgemeinen Formel II

**R**^{**3**}**O(-Z)**_{**x**} (II)

eingesetzt,
worin R³ für einen linearen oder verzweigten C₆₋₂₂-Alkylrest, Z für ein beliebiges Mono- oder Oligosaccharid und x für einen Wert von 1 bis 5 steht. Vorzugsweise steht R³ für lineare oder in 2-Stellung methylverzweigte Alkylreste. Solche Alkylreste R³ sind beispielsweise 1-Octyl-, 1-Decyl-, 1-Lauryl-, 1-Myristyl-, 1-Cetyl- und 1-Stearylreste. Besonders bevorzugt sind 1-Octyl-, 1-Decyl-, 1-Lauryl- oder 1-Myristylreste. Bei Verwendung sogenannter *"Oxo-Alkohole"* als Ausgangsstoffe zur Herstellung der Verbindungen der allgemeinen Formel II überwiegen Alkylreste R³ mit einer ungeraden Anzahl von Kohlenstoffatomen in der Alkylkette.

Die als Komponente B verwendbaren Alkylpolyglykoside können beispielsweise nur einen Übestimmten Alkylrest R³ enthalten. Üblicherweise werden die Alkylpolyglycoside aber ausgehend von natürlichen Fetten und Ölen oder Mineralölen hergestellt. In diesem Fall liegen als Alkylreste R³ Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R³
- im wesentlichen für C₈- und C₁₀-Alkylgruppen,
- im wesentlichen für C₁₂- und C₁₄-Alkylgruppen,
- im wesentlichen für C₈- bis C₁₆-Alkylgruppen oder
- im wesentlichen für C₁₂- bis C₁₆-Alkylgruppen
   steht.

Der Zuckerbaustein Z leitet sich von beliebigen Mono- oder Oligosacchariden ab. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose. Besonders bevorzugt ist Glucose.

Die erfindungsgemäß verwendbaren Alkylpolyglykoside enthalten pro Alkylglykosideinheit im Schnitt etwa 1,1 bis etwa 5 Zuckereinheiten. Alkylpolyglykoside mit x-Werten von etwa 1,2 bis etwa 1,8 sind bevorzugt. Ganz besonders bevorzugt sind Alkylpolyglykoside, bei denen x etwa 1,3 bis etwa 1,6 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Ebenfalls zum Einsatz in den erfindungsgemäßen manuellen Reinigungsmitteln oder insbesondere HGSM geeignet sind Tenside aus der Familie der Glucamide, beispielsweise Alkyl-N-Methylglucamide, wobei der Begriff Alkyl sich auf Alkylreste mit einer Kettenlänge von etwa 6 bis etwa 14 Kohlenstoffatomen bezieht. Es kann vorteilhaft sein, wenn die beschriebenen nichtionischen Tenside nicht als alleiniger Bestandteil der Komponente B, sondern jeweils im Gemisch mit mindestens einem weiteren nichtionischen Tensid eingesetzt werden, z.B. in Kombination mit Fettalkoholethoxylat und/oder Glucamid. Gegebenenfalls sind auch quartäre oder noch höhere Kombinationen der hier offenbarten nichtionischen Tenside möglich.

Das erfindungsgemäße Tensidgemisch enthält die Komponente B gegebenenfalls in einem Anteil von bis zu etwa 99 Gew.-%, vorzugsweise in einem Anteil von etwa 10 Gew.-% bis etwa 80 Gew.-%. Besonders bevorzugt sind Gehalte von etwa 15 Gew.-% bis etwa 55 Gew.-%, beispielsweise etwa 20 bis etwa 40 Gew.-%.

Das Verhältnis von Komponente A zu Komponente B im erfindungsgemäßen Reinigungsmittel, insbesondere HGSM, beträgt in der Regel etwa 1:4 bis etwa 4:1, bevorzugt etwa 1:2 bis etwa 2:1.

Als Komponente C enthält das erfindungsgemäße Tensidgemisch gegebenenfalls mindestens ein anionisches Tensid, das kein Gemini-Tensid ist.

Typische Beispiele für im Rahmen der Erfindung einsetzbare anionische Tenside sind Alkylbenzolsulfonate, Alkansulfonate, Olefinsulfonate, Alkylethersulfonate, Glycerinethersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Dialkylsulfosuccinate, Mono- und Dialkylsulfosuccinamate Sulfotriglyceride, Amidseifen, Ethercarbonsäuren und deren Salze, Fettsäureisothionate, Fettsäuresarkosinate, Fettsäuretauride, Acyllactate, Acyloligoglykosidsulfate, Proteinfettsäurekondensate (insbesondere pflanzliche Produkte auf Sojabasis) oder Alkyl(ether)phosphate oder Gemische aus zwei oder mehr davon.

Vorzugsweise enthält das erfindungsgemäße Tensidgemisch als Komponente C mindestens ein anionisches Tensid ausgewählt aus der Gruppe der Alkylsulfate, Alkylethersulfate, Alkansulfonate, Alkylbenzolsulfonate oder Olefinsulfonate.

Besonders bevorzugt als Komponente C, oder zumindest als Bestandteil der Komponente C, sind im Rahmen der vorliegenden Erfindung anionische Tenside aus der Gruppe der Alkylsulfate. Hierzu zählen beispielsweise Tenside der allgemeinen Formel III 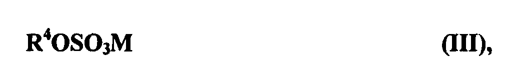 worin R⁴ für einen aliphatischen, linearen und/oder verzweigten C₆₋₂₂-Alkylrest und M für ein Metallkation oder Ammoniumion steht.

Ebenfalls als anionische Tenside im Rahmen der Erfindung einsetzbar sind Fettalkoholethersulfate, die großtechnisch durch SO₃- oder Chlorsulfonsäure(CSA)-Sulfatierung von Fettalkoholpolyalkylenethern und nachfolgender Neutralisation hergestellt werden. Typische Beispiele sind die Sulfate von Anlagerungsprodukten von durchschnittlich 1 - 10 und insbesondere 2 - 5 mol Ethylenoxid an Capronalkohol, Caprylalkohol, 2-Ethylhexylalkohol, Caprinalkohol, Laurylalkohol, Isotridecylalkohol, Myristylalkohol, Palmoleyl-alkohol, Stearylalkohol, Isostearylalkohol, Oleylalkohol, Elaidylalkohol, Petroselinylalkohol, Arachylalkohol, Gadoleylalkohol, Behenylalkohol, Erucyl-alkohol, und Brassidylalkohol sowie deren technische Mischungen, wobei die Sulfate in der Regel als Alkalisalz, vorzugsweise als Natriumsalz oder als Erdalkalimetallsalz, vorzugsweise als Magnesiumsalz, oder als Gemische aus zwei oder mehr der letztgenannten Salze, eingesetzt werden.

Das erfindungsgemäße Tensidgemisch enthält die Komponente C in einem Anteil von bis zu etwa 99 Gew.-%, vorzugsweise bis zu etwa 80 Gew.-%. Besonders bevorzugt ist ein Gehalt an Komponente C von etwa 5 Gew.-% bis etwa 70 Gew.-%, wobei ein Gehalt von etwa 20 Gew.-% bis etwa 60 Gew.-% bevorzugt ist.

Das erfindungsgemäße Tensidgemisch enthalt damit bevorzugt
- etwa 0,1 bis etwa 99 Gew.-% Komponente A,
- etwa 0 bis etwa 99 Gew.-% Komponente B und
- etwa 0 bis etwa 99 Gew.-% Komponente C,
wobei die Summe der Anteile von A, B und C 100 Gew.-% ergibt.

Das erfindungsgemäße Tensidgemisch muß jeweils mindestens eine Kombination der Komponenten A und B oder A und C enthalten. Im ersten Fall enthalt ein bevorzugtes Tensidgemisch etwa 20 Gew.-% bis etwa 45 Gew.-% der Komponente A und etwa 80 bis etwa 55 Gew.-% der Komponente B. Im letzten Fall enthält das Tensidgemisch etwa 10 Gew.-% bis etwa 35 Gew.-% der Komponente A und etwa 15 Gew.-% bis etwa 50 Gew.-% der Komponente C.

In einer bevorzugten Ausführungsform enthält das erfindungsgemäße Tensidgemisch jedoch alle drei Komponenten A, B und C. In diesem Fall sind beispielsweise Tensidgemische der folgenden Zusammensetzung bevorzugt:
- Komponente A:: etwa 20 Gew.-% bis etwa 50 Gew.-%
- Komponente B:: etwa 15 Gew.-% bis etwa 35 Gew.-%
- Komponente C:: etwa 20 Gew.-% bis etwa 60 Gew.-%
oder
- Komponente A:: etwa 15 Gew.-% bis etwa 35 Gew.-%
- Komponente B:: etwa 30 Gew.-% bis etwa 70 Gew.-%
- Komponente C:: etwa 1 Gew.-% bis etwa 45 Gew.-%.

In einer bevorzugten Ausführungsform der Erfindung ergibt die Kombination der Komponenten A, B und C zusammen 100 Gew.-%.

Die beschriebenen Tensidgemische können in Reinigungsmitteln, insbesondere in HGSM eingesetzt werden.

Gegenstand der Erfindung ist daher auch ein Reinigungsmittel, insbesondere ein HGSM, enthaltend ein Tensidgemisch, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
   und
b) 0 bis 99 Gew.-% mindestens eines nichtionischen Tensids, das kein Gemini-Tensid ist, als Komponente B
   und/oder
c) 0 bis 99 Gew.-% mindestens eines anionischen Tensids, das kein Gemini-Tensid ist, als Komponente C,
   wobei die Summe der Anteile der Komponenten A, B und C 100 Gew.-% ergibt Zusätzlich zu den Komponenten A, B und C kann das erfindungsgemäße Mittel noch weitere Zusatzstoffe, beispielsweise Lösemittel, Builder, Abrasivstoffe, Farbstoffe und/oder Duftstoffe enthalten.

Als Lösemittel kann das erfindungsgemäße Mittel beispielsweise Wasser oder niedere Mono- oder Polyalkohole, beispielsweise Ethanol, Isopropanol, Ethylenglykol, Propylenglykol, Alkanolamine oder Glycerin enthalten. Die Lösemittel können, bezogen auf das gesamte Mittel, in einer Menge von bis zu 50 Gew.-%, vorzugsweise jedoch darunter, im erfindungsgemäßen Mittel enthalten sein.

Unter Buildern werden im Sinne der vorliegenden Erfindung Verbindungen verstanden, die zentrale Aufgaben im Reinigungsprozeß übernehmen. Sie tragen nicht nur durch spezifische Effekte selbst zum Waschgeschehen bei, sondern beeinflussen auch in entscheidender Weise die Wirkung der anderen Reinigungsmittelkomponenten, z.B. von Tensiden oder Bleichmitteln. Zu den von Buildern bewirkten Effekten zählt z.B. die Eliminierung von sogenannten "Härtebildnern" Reinigungsflotte und Reinigungsgut sowie die Dispergierung von glöstem Schmutz und anderen Schwebstoffen.

Als gegebenenfalls einsetzbare Builder eignen sich solche aus der Klasse der Aminopolycarbonsäuren und Polyphosphonsäuren. Zu den Aminopolycarbonsäuren zählen Nitrilotriessigsäure, Ethylendiamintetraessigsäure, Diethylentriaminpentaessigsäure sowie deren höhere Homologen. Geeignete Polyphosphonsäuren sind 1-Hydroxyethan-1,1-diphosphonsäure, Aminotri(methylenphosphonsäure), Ethylendiamintetra(methylenphosphonsäure) und deren höhere Homologen, wie Diethylentetramintetra(methylenphosphonsäure). Die genannten Säuren kommen üblicherweise in Form ihrer Alkalisalze, insbesondere der Natrium- bzw. Kaliumsalze zur Anwendung. Bevorzugt wird Natriumnitrilotriacetat in Anteilen bis zu 10 Gew.-%, vorzugsweise 2 Gew.-% bis 6 Gew.-%, bezogen auf das Reinigungsmittel, oder insbesondere HGSM, eingesetzt.

Zu den geeigneten Buildern gehören ferner monomere Polycarbonsäuren bzw. Hydroxypolycarbonsäuren, insbesondere in Form der Alkalisalze, beispielsweise Natriumcitrat und/oder Natriumgluconat.

Zu den bevorzugt eingesetzten Buildern zählen homopolymere und/oder copolymere Carbonsäuren bzw. deren Alkalisalze, wobei die Natrium- oder Kaliumsalze bevorzugt sind. Besonders geeignet sind polymere Carboxylate beziehungsweise polymere Carbonsäuren, mit einem relativen Molekulargewicht Mₙ von mindestens 350, in Form ihrer wasserlöslichen Salze, insbesondere in Form der Natrium- und/oder Kaliumsalze, beispielsweise oxidierte Polysaccharide gemäß der WO-A 93/08251, Polyacrylate, Polyhydroxyacrylate, Polymethacrylate, Polymaleate und insbesondere Copolymere der Acrylsäure mit Maleinsäure bzw. Maleinsäureanhydrid, vorzugsweise solche aus 50 bis 70 Gew.-% Acrylsäure und 50 bis 10 Gew.-% Maleinsäure, wie sie z.B. in der EP-A 0 022 551 charakterisiert sind. Das relative Molekulargewicht Mₙ der Homopolymeren liegt im allgemeinen zwischen 1.000 und 100.000, die der Copolymeren zwischen 2.000 und 200.000, vorzugsweise 50.000 bis 120.000, bezogen auf freie Säure.

Besonders bevorzugte Acrylsäure-Maleinsäure-Copolymere weisen eine relatives Molekulargewicht Mₙ von 50.000 bis 100.000 auf.

Geeignete, wenn auch weniger bevorzugte Verbindungen dieser Klasse sind Copolymere der Acrylsäure und/oder Methacrylsäure mit Vinylethern, wie Vinylmethylethern, Vinylestern, Ethylen, Propylen und/oder Styrol, in denen der Anteil der jeweiligen Säure, oder der Anteil eines Gemischs der Säuren, mindestens 50 Gew.-% beträgt.

Als polymere Carboxylate beziehungsweise Carbonsäuren können auch Terpolymere eingesetzt werden, die als Monomere zwei Carbonsäuren und/oder deren Salze sowie als drittes Monomer Vinylalkohol und/oder ein Vinylalkoholderivat oder ein Kohlehydrat enthalten. Das erste saure Monomer bzw. dessen Salz leitet sich von einer monoethylenisch ungesättigten C₃₋₈-Carbonsäure und vorzugsweise von einer C₃₋₄-Monocarbonsäure, insbesondere von der (Meth)acrylsäure ab. Das zweite saure Monomer bzw. dessen Salz kann ein Derivat einer C₄₋₈-Dicarbonsäure, vorzugsweise einer C₄₋₈-Dicarbonsäure sein, wobei Maleinsäure bevorzugt ist. Die dritte monomere Einheit wird in diesem Fall von Vinylalkohol und/oder vorzugsweise einem veresterten Vinylalkohol gebildet. Insbesondere sind Vinylalkoholderivate bevorzugt, welche einen Ester aus kurzkettigen Carbonsäuren, beispielsweise von C₁₋₄-Carbonsäuren, mit Vinylalkohol darstellen. Bevorzugte Terpolymere enthalten dabei 60 bis 95 Gew.-%, insbesondere 70 bis 90 Gew.-% (Meth)acrylsäure bzw. (Meth)acrylat, besonders bevorzugt Acrylsäure bzw. Acrylat, und Maleinsäure bzw. Maleat sowie 5 bis 40 Gew.-%, vorzugsweise 10 bis 30 Gew.-% Vinylalkohol und/oder Vinylacetat. Ganz besonders bevorzugt sind dabei Terpolymere, in denen das Gewichtsverhältnis (Meth)acrylsäure bzw. (Meth)acrylat zu Maleinsäure bzw. Maleat zwischen 1:1 und 4:1, vorzugsweise zwischen 2:1 und 3:1 und insbesondere zwischen 2:1 und 2,5:1 liegt. Dabei sind sowohl die Mengen- als auch die Gewichtsverhältnisse auf die Säuren bezogen. Das zweite saure Monomer bzw. dessen Salz kann auch ein Derivat einer Allylsulfonsäure sein, die in 2-Stellung mit einem Alkylrest, vorzugsweise mit einem C₁₋₄-Alkylrest, oder einem aromatischen Rest, der sich vorzugsweise von Benzol oder Benzolderivaten ableitet, substituiert ist.

Bevorzugte Terpolymere enthalten dabei etwa 40 bis 60 Gew.-%, insbesondere etwa 45 bis 55 Gew.-% (Meth)acrylsäure bzw. (Meth)acrylat, besonders bevorzugt Acrylsäure bzw. Acrylat, etwa 10 bis 30 Gew.-%, vorzugsweise etwa 15 bis 25 Gew.-% Methallylsulfonsäure bzw. Methallylsulfonat und als drittes Monomer etwa 15 bis 40 Gew.-%, vorzugsweise etwa 20 bis 40 Gew.-% eines Kohlehydrats. Dieses Kohlehydrat kann dabei beispielsweise ein Mono-, Di-, Oligo- oder Polysaccharid sein, wobei Mono-, Di- oder Oligosaccharide bevorzugt sind, besonders bevorzugt ist Saccharose. Durch den Einsatz dieses dritten Monomeren werden Sollbruchstellen im Polymeren eingebaut, die für die biologische Abbaubarkeit des Polymeren verantwortlich sind. Die eingesetzten Terpolymeren lassen sich nach den bekannten und üblichen Verfahren herstellen. Bevorzugt werden auch solche Terpolymeren eingesetzt, die entweder vollständig, oder zumindest partiell, insbesondere zu mehr als etwa 50 %, bezogen auf die vorhandenen Carboxylgruppen, neutralisiert sind.

Brauchbar sind ferner Polyacetalcarbonsäuren, wie sie beispielsweise durch Polymerisation von Estern der Glykolsäure, Einführung stabiler terminaler Endgruppen und Verseifing zu den Natrium- oder Kaliumsalzen, erhalten werden. Geeignet sind ferner polymere Säuren, wie sie durch Polymerisation von Acrolein und Disproportionierung des Polymeren nach Canizzaro mittels starker Alkalien erhältlich sind. Sie sind im wesentlichen aus Acrylsäureeinheiten und Vinylalkoholeinheiten beziehungsweise Acroleineinheiten aufgebaut.

Der Anteil an organischen, carboxylgruppenhaltigen Buildern im erfindungsgemäßen Mittel kann bis zu etwa 10 Gew.-%, vorzugsweise etwa 1 Gew.-% bis etwa 7,5 Gew.-% und insbesondere etwa 2 Gew.-% bis 5 Gew.-%, der Gehalt an Polyphosphonsäuren bis zu etwa 3 Gew.-%, vorzugsweise etwa 0,05 Gew.-% bis etwa 1,5 Gew.-%, insbesondere etwa 0,1 Gew.-% bis etwa 1 Gew.-%, betragen.

Brauchbare Builder sind ferner kristalline Alkalisilikate sowie feinteilige Alkalialumosilikate, insbesondere Zeolithe vom Typ NaA. Geeignete Zeolithe weisen ein Calciumbindevermögen im Bereich von 100 bis 200 mg CaO/g (gemäß den Angaben in der DE-C 24 12 837) auf. Ihre Teilchengröße liegt üblicherweise im Bereich von 1 µm bis 10 µm. Sie kommen in trockener Form zum Einsatz. Das in den Zeolithen in gebundener Form enthaltene Wasser stört im vorliegenden Falle nicht. Als kristalline Silikate, die allein oder im Gemisch mit den genannten Alumosilikaten vorliegen können, werden vorzugsweise kristalline Schichtsilikate der Formel NaMSiₓ-O₂ₓ₊₁·yH₂O eingesetzt, in denen M für Natrium steht, x eine Zahl von 1,9 bis 4 und y eine Zahl von 0 bis 20 ist und bevorzugte Werte für x 2, 3 oder 4 sind. Derartige kristalline Schichtsilikate werden beispielsweise in der EP-A 0 164 514 beschrieben. Insbesondere sind sowohl β- als auch ä-Natriumdisilikate Na₂Si₂O₅·yH₂O bevorzugt, wobei β-Natriumdisilikat beispielsweise nach dem Verfahren erhalten werden kann, das in der WO-A 91/08171 beschrieben ist. Brauchbare kristalline Silikate sind unter den Bezeichnungen SKS-6 (Hersteller Hoechst) und Nabion® 15 (Hersteller Rhône-Poulenc) im Handel. Der Gehalt an anorganischen Gerüstsubstanzen am manuellen Reinigungsmittel oder HGSM kann bis zu etwa 35 Gew.-%, vorzugsweise bis zu etwa 25 Gew.-% und insbesondere etwa 10 Gew.-% bis etwa 25 Gew.-% betragen.

Die erfindungsgemäßen Reinigungsmittel, insbesondere HGSM, sind vorzugsweise phosphatfrei. Sofern ein Phosphatgehalt ökologisch unbedenklich ist (zum Beispiel bei einer Phosphate eliminierenden Abwasserreinigung), können auch polymere Alkaliphosphate, wie Natriumtripolyphosphat, anwesend sein. Ihr Anteil kann bis zu etwa 20 Gew.-%, bezogen auf das gesamte Mittel, betragen, wobei der Anteil der übrigen Feststoffe, zum Beispiel des Alkalisilikats und/oder Alumosilikats, entsprechend vermindert wird. Vorzugsweise beträgt der Anteil an Tripolyphosphat höchstens 10 Gew.-%.

Die genannten Builder können im erfindungsgemäßen Mittel einzeln, oder aber als Gemisch aus zwei oder mehr davon vorliegen. Der Gesamtgehalt der erfindungsgemäßen Mittel an Builder oder einem Gemisch aus zwei oder mehr Buildern beträgt bis zu etwa 5 Gew.-%, vorzugsweise von etwa 0,1 Gew.-% bis etwa 3 Gew.-% und besonders bevorzugt von etwa 0,5 Gew.-% bis etwa 1,5 Gew.-%.

Das erfindungsgemäße Reinigungsmittel kann in einer Menge von bis zu etwa 70 Gew.-% einen Abrasivstoff oder ein Gemisch aus mehreren Abrasivstoffen enthalten. Der Abrasivstoff ist vorzugsweise feinteilig, mit einer mittleren Korngröße im Bereich von etwa 5 µm bis 100 µm, wobei höchstens 10 % der Teilchen eine Korngröße von mehr als 150 µm aufweisen. Vorzugsweise beträgt die mittlere Korngröße der die feste Phase bildenden Teilchen 10 µm bis 80 µm und insbesondere 10 µm bis 60 µm, wobei die maximale Korngröße unterhalb 200 µm, insbesondere unter 150 µm liegt. Die mittlere Korngröße bezieht sich auf die Volumen-Verteilung der Teilchen, die nach bekannten Methoden (beispielsweise mittels Coulter Counter) bestimmt werden kann.

Als Abrasivstoff kann ein wasserunlöslicher oder ein wasserlöslicher Stoff eingesetzt werden. Zu den wasserunlöslichen Abrasivstoffen, die im Rahmen der vorliegenden Erfindung eingesetzt werden können, zählen beispielsweise Quarzmehl, Marmormehl, Kreide, Bimsstein, Schichtsilikate und/oder Feldspat.

Wasserlösliche Abrasivstoffe sind in der Regel wasserlösliche Salze, ausgewählt aus der Gruppe der Chloride, Carbonate, Hydrogencarbonate, Sulfate, Phosphate, Borate oder Silicate. Vorzugsweise handelt es sich dabei um Alkalimetallsalze, besonders bevorzugt um die Salze von Natrium und/oder Kalium.

Wasserlösliche Abrasivstoffe werden vorzugsweise bei sogenannten "two-in-one" Produkten eingesetzt, die sowohl in konzentrierter als auch in verdünnter Form eingesetzt werden können. Der Einsatz von wasserlöslichen Abrasivstoffen in wasserhaltigen Mitteln ist zwar möglich wenn die Menge an zugesetztem Abrasivstoff das abrasivstoffbezogene Lösungsvermögen des Wassers überschreitet, aber nicht bevorzugt.

Abrasivstoffe können im erfindungsgemäßen Mittel einzeln, oder als Gemisch von zwei oder mehr Abrasivstoffen, in einem Anteil von bis zu etwa 70 Gew.-%, vorzugsweise von etwa 30 Gew.-% bis etwa 65 Gew.-% und besonders bevorzugt von etwa 40 Gew.-% bis zu etwa 60 Gew.-% vorliegen.

Farbstoffe und Duftstoffe können im erfindungsgemäßen Mittel in untergeordneten Mengen von insgesamt bis zu etwa 3 Gew.-% vorliegen.

Das erfindungsgemäße Reinigungsmittel kann u.a. als Feststoff, als Paste oder als Flüssigkeit formuliert sein und enthält das erfindungsgemäße Tensidgemisch in einer Menge von bis zu etwa 99 Gew.-%. Bevorzugt enthält das erfindungsgemäße Mittel das erfindungsgemäße Tensidgemisch in einer Menge von etwa 3 Gew.-% bis etwa 50 Gew.-%, besonders bevorzugt in einer Menge von 5 Gew.-% bis etwa 30 Gew.-%.

Beispielhafte Formulierungen für erfindungsgemäßen Reinigungsmittel enthalten etwa
- 9 Gew.-% Gemini-Tensid (Dimerdiolethersulfat-Natriumsalz, 20 EO)
- 6 Gew.-% APG 600® (Henkel, Düsseldorf)
- 3 Gew.-% Kokosamidopropylbetain
- 6 Gew.-% C₁₂₋₁₄-Fettalkoholsulfat-Natriumsalz
oder
- 13 Gew.-% Gemini-Tensid (Dimerdiolethersulfat-Natriumsalz, 20 EO)
- 5 Gew.-% APG 600® (Henkel, Düsseldorf)
- 2 Gew.-% APG 220® (Henkel, Düsseldorf)
- 4 Gew.-% Kokosamidopropylbetain
- 8 Gew.-% C₁₂₋₁₄-Fettalkoholsulfat-Natriumsalz
- 1,5 Gew.-% Carbopol 880® (B.F. Goodrich, OH, USA)
oder
- 4 Gew.-% Gemini-Tensid (Dimerdiolethersulfat-Natriumsalz, 20 EO)
- 3 Gew.-% Dehydol 980® (Henkel, Düsseldorf)
- 1,5 Gew.-% C₁₂₋₁₄-Fettalkoholsulfat-Natriumsalz
- 0,5 Gew.-% Natriumgluconat
- 1 Gew.-% C₁₂₋₁₄-Fettsäure-Natriumsalz,
wobei der zu 100 Gew.-% fehlende Anteil aus jeweils weiteren im Rahmen des vorliegenden Textes erwähnten Zusatzstoffen und/oder Lösemitteln, vorzugsweise Wasser, besteht.

Ebenfalls Gegenstand der Erfindung ist die Verwendung eines Tensidgemischs, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
   und
b) 0 bis 99 Gew.-% mindestens eines nichtionischen Tensids, das kein Gemini-Tensid ist, als Komponente B
   und/oder
c) 0 bis 99 Gew.-% mindestens eines anionischen Tensids, das kein Gemini-Tensid ist, als Komponente C,
   wobei die Summe der Anteile der Komponenten A, B und C 100 Gew.-% ergibt, in einem Reinigungsmittel, insbesondere in einem HGSM.

Die nachfolgenden Beispiele sollen die Erfindung näher erläutern, sie üben jedoch keine beschränkende Wirkung aus.

### Beispiele:

Die folgenden Verbindungen wurden als Komponente A, B und C eingesetzt:
- Komponente A:: Dimerdiolethersulfat-Natriumsalz (20 EO)
- Komponente B:: C₁₂₋₁₄-Alkylpolyglycosid mit einem Oligomerisierungsgrad von etwa 1,5
- Komponente C:: C₁₂₋₁₄-Alkylsulfat-Natriumsalz

Als Laborstandard diente ein Tensidgemisch, bestehend aus
- 60 Gew.-% Alkylbenzolsulfonat-Natriumsalz
- 40 Gew.-% C₁₂₋₁₄-Fettalkoholsulfat-2EO-Natriumsalz
als wäßrige Lösung mit insgesamt 10 Gew.-% Aktivsubstanz.

### Beispiel 1:

40 Gew.-% Komponente A und 60 Gew.-% einer 50 Gew.-%igen wäßrigen Lösung von Komponente B wurden zu einem Vorgemisch V verrührt. Dieses Vorgemisch V wurde anschließend in den in der Tabelle 1 angegebenen Mischungsverhältnissen mit Komponente C vermischt und mit Wasser aufeinen Aktivsubstanzgehalt von 10 Gew.-% verdünnt.

Von den so erhältlichen erfindungsgemäßen Gemischen wurden anschließend jeweils 0,4g (AVO-Anschmutzung) bzw. 0,5g (Rita-Anschmutzung) in etwa 10 l Wasser mit einer Temperatur von etwa 40°C (AVO-Anschmutzung) bzw. etwa 45°C (Rita-Anschmutzung) gegeben. Anschließend wurden vorher entsprechend angeschmutzte Untertassen solange in dieser Flotte gereinigt, bis die Schaumdecke die Spülflotte nur noch zu 50% bedeckte.

Zum Vergleich wurden identische Mengen des Laborstandards in 10 l Wasser mit einer zu den o.A. Versuchen jeweils identischen Temperatur von 40°C bzw. 45°C gegeben und die Spülprozedur damit wiederholt. Die Ergebnisse sind der Tabelle 1 zu entnehmen.

**Tabelle 1**

| Mischungsverhältnis | | Spülergebnis (% des Laborstandards) | |
|---|---|---|---|
| V | C | Rita | AVO |
| 100 | 0 | 77 | 97 |
| 80 | 20 | 108 | 127 |
| 60 | 40 | 115 | 121 |
| 40 | 60 | 87 | 118 |
| 20 | 80 | 62 | 97 |
| 0 | 100 | 38 | 36 |
| Rita = Rindertalganschmutzung AVO = Anschmutzung, basierend auf Schweineschmalz und Olivenöl | | | |

### Beispiel 2:

- 20 Gew.-% Komponente: A und 80 Gew.-% einer 50 Gew.-%igen wäßrigen Lösung von Komponente B wurden zu einem Vorgemisch V verrührt. Dieses Vorgemisch V wurde anschließend in den in der Tabelle 1 angegebenen Mischungsverhältnissen mit Komponente C vermischt und mit Wasser auf einen Aktivsubstanzgehalt von 10 Gew.-% verdünnt. Von den so erhältlichen erfindungsgemäßen Gemischen wurden anschließend jeweils wieder 0,4 g (AVO) bzw 0,5 g (Rita) in etwa 10 l Wasser mit einer Temperatur von etwa 40°C (AVO) bzw. 45°C (Rita) gegeben. Anschließend wurden vorher entsprechend angeschmutzte Untertassen solange in dieser Flotte gereinigt, bis die Schaumdecke die Spülflotte nur noch zu 50% bedeckte.
Nachfolgend wurde wieder mit dem Laborstandard verglichen, die Ergebnisse sind in Tabelle 2 zusammengefaßt.

**Tabelle 2**

| Mischungsverhältnis | | Spülergebnis (% des Laborstandards) | |
|---|---|---|---|
| V | C | Rita | AVO |
| 100 | 0 | 100 | 142 |
| 80 | 20 | 123 | 155 |
| 60 | 40 | 123 | 152 |
| 40 | 60 | 92 | 133 |
| 20 | 80 | 69 | 106 |
| 0 | 100 | 38 | 39 |
| Rita = Rindertalganschmutzung AVO = Anschmutzung, basierend auf Schweineschmalz und Olivenöl | | | |

## Patentansprüche

1. Verbindung der allgemeinen Formel I 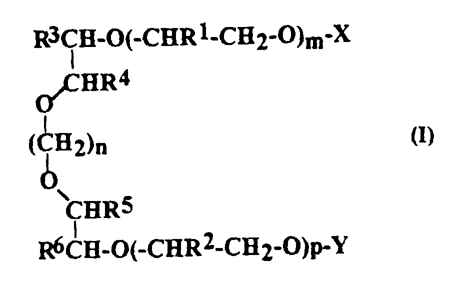 worin R¹ und R² unabhängig voneinander für Wasserstoff und/oder C₁₋₆-Alkyl, R³, R⁴, R⁵ und R⁶ unabhängig voneinander für Wasserstoff oder lineare oder verzweigte Alkylreste mit 4 bis 24 C-Atomen, n für Werte von 2 bis 10, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen oder für quaternisierte Stickstoffbasen steht, wobei jeweils einer der beiden Reste der Restepaare R³ und R⁴ sowie R⁵ und R⁶ für Wasserstoff stehen muß.

2. Tensidgemisch, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
und
b) 0 bis 99 Gew.-% mindestens eines nichtionischen Tensids, das kein Gemini-Tensid ist, als Komponente B
und/oder
c) 0 bis 99 Gew.-% mindestens eines anionischen Tensids, das kein Gemini-Tensid ist, als Komponente C,
wobei die Summe der Anteile der Komponenten A, B und C 100 Gew.-% ergibt.

3. Tensidgemisch nach Anspruch 1, dadurch gekennzeichnet, daß es als Komponente A mindestens ein Dimeralkohol-bis-ethersulfat und/oder ein Trimeralkohol-tris-ethersulfat enthält, das erhältlich ist, indem die Anlagerungsprodukte von Dimer- und/oder Trimeralkoholen und Alkylenoxiden oder ein Gemisch solcher Anlagerungsprodukte mit einem Sulfiermittel umgesetzt werden und anschließend mit einer wäßrigen Base neutralisiert werden.

4. Tensidgemisch nach Anspruch 2, dadurch gekennzeichnet, daß es als Komponente A mindestens eine Verbindung der allgemeinen Formel I enthält, 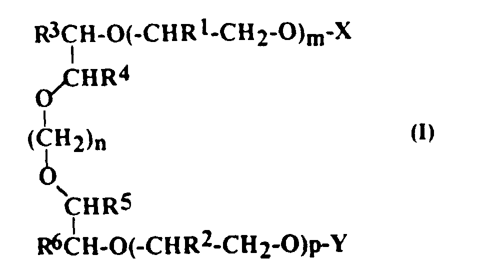 worin R¹ und R² unabhängig voneinander für Wasserstoff und/oder C₁₋₆-Alkyl, R³, R⁴, R⁵ und R⁶ unabhängig voneinander für Wasserstoff oder lineare oder verzweigte Alkylreste mit 4 bis 24 C-Atomen, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen oder für quaternisierte Stockstoffbasen steht, wobei bei den Restepaaren R³ und R⁴ sowie R⁵ und R⁶ jeweils einer der beiden Reste für Wasserstoff stehen muß.

5. Tensidgemisch nach Anspruch 4, dadurch gekennzeichnet, daß R¹ und R² für Wasserstoff, R³ oder R⁴ für einen unverzweigten Alkylrest mit mehr als 10 Kohlenstoffatomen, wobei der jeweils verbleibende Rest für Wasserstoff steht, R⁵ oder R⁶ für einen unverzweigten Alkylrest mit mehr als 10 Kohlenstoffatomen, wobei der jeweils verbleibende Rest für Wasserstoff steht, und X und Y für SO₃Na stehen.

6. Tensidgemisch nach Anspruch 2 bis 5, dadurch gekennzeichnet, daß es als Komponente B mindestens eine Verbindung der allgemeinen Formel II enthält,
R³O(-Z)ₓ (II)
worin R³ für einen linearen oder verzweigten C₆₋₂₂-Alkylrest, Z für einen beliebigen Mono- oder Oligosaccharidbaustein und x für einen Wert von 1 bis 5 steht.

7. Tensidgemisch nach Anspruch 2 bis 6, dadurch gekennzeichnet, daß es als Komponente C mindestens ein anionisches Tensid, ausgewählt aus der Gruppe der Alkylsulfate, Alkylethersulfate, Alkansulfonate, Alkylbenzolsulfonate oder Olefinsulfonate enthält.

8. Tensidgemisch nach Anspruch 2 bis 7, dadurch gekennzeichnet, daß es als Komponente C mindestens ein Alkylsulfat enthält.

9. Tensidgemisch nach Anspruch 2 bis 8, dadurch gekennzeichnet, däß es
- 0,1 bis 99 Gew.-% Komponente A,
- 0 bis 99 Gew.-% Komponente B und
- 0 bis 99 Gew.-% Komponente C
enthält.

10. Verwendung eines Tensidgemischs, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
und
b) 0 bis 99 Gew.-% mindestens eines nichtionischen Tensids, das kein Gemini-Tensid ist, als Komponente B
und/oder
c) 0 bis 99 Gew.-% mindestens eines anionischen Tensids, das kein Gemini-Tensid ist, als Komponente C,
wobei die Summe der Anteile der Komponenten A, B und C 100 Gew.-% ergibt, in Reinigungsmitteln, insbesondere in HGSM.

11. Reinigungsmittel, insbesondere HGSM, enthaltend ein Tensidgemisch, enthaltend
a) 0,1 Gew.-% bis 99 Gew.-% mindestens eines Tensids mit mindestens zwei hydrophilen und mindestens zwei hydrophoben Gruppen, wobei die hydrophilen Gruppen und die hydrophoben Gruppen jeweils untereinander durch einen mindestens vier C-Atome enthaltenden, linearen oder cyclischen Spacer getrennt sind (Gemini-Tensid), als Komponente A,
und
b) 0 bis 99 Gew.-% mindestens eines nichtionischen Tensids, das kein Gemini-Tensid ist, als Komponente B
und
c) 0 bis 99 Gew.-% mindestens eines anionischen Tensids, das kein Gemini-Tensid ist, als Komponente C,
wobei die Summe der Anteile der Komponenten A, B und C 100 Gew.-% ergibt.

12. Reinigungsmittel, insbesondere HGSM, nach Anspruch 11, dadurch gekennzeichnet, daß das Tensidgemisch in einer Menge von 3 Gew.-% bis 50 Gew.-% enthalten ist.

13. Verwendung von Verbindungen der allgemeinen Formel I 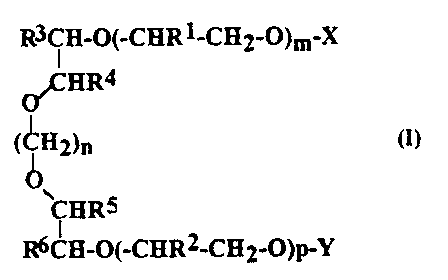 worin R¹ und R² unabhängig voneinander für Wasserstoff und/oder C₁₋₆-Alkyl, R³, R⁴, R⁵ und R⁶ unabhängig voneinander für Wasserstoff oder lineare oder verzweigte Alkylreste mit 4 bis 24 C-Atomen, n für Werte von 2 bis 10, m und p unabhängig voneinander für Werte von 1 bis 40, X und Y unabhängig voneinander für Wasserstoff, CH₂COOM oder SO₃M und M für Wasserstoff oder Alkali- oder Erdalkalimetallionen stehen, wobei jeweils einer der beiden Reste der Restepaare R³ und R⁴ sowie R⁵ und R⁶ für Wasserstoff stehen muß, in Waschmitteln, Reinigungsmitteln für harte Oberflächen, Körperreinigungsmitteln, Kosmetika, maschinellen Geschirrspülmitteln, Handgeschirrspülmitteln, als technische Emulgatoren, Netzmittel, Dispergatoren, insbesondere in Lacken und Farben, Demulgatoren, Hydrotrope, Antistatika, Additive für die Erdölindustrie, Kraftstoffadditive, oder in der Metall und Textilverarbeitung.
